# EUROPEAN PATENT APPLICATION

(11) **EP 4 434 433 A1**
(43) Date of publication of application: **25.09.2024**
(21) Application number: 21964731.0
(22) Date of filing: 18.11.2021
(51) Int. Cl.: A61B 1/045

(54) **INFORMATION DISPLAY DEVICE, INFORMATION DISPLAY METHOD, AND RECORDING MEDIUM**

(71) Applicant: NEC Corporation, 108-8001 Tokyo (JP)
(72) Inventor: KAMIJO, Kenichi, Tokyo 108-8001 (JP); TAKAHASHI, Ikuma, Tokyo 108-8001 (JP); OKUTSU, Motoyasu, Tokyo 108-8001 (JP); OHTSUKA, Shota, Tokyo 108-8001 (JP); KIMURA, Tatsu, Tokyo 108-8001 (JP)
(74) Representative: Betten & Resch
(86) International application number: PCT/JP2021/042365
(87) International publication number: WO 2023/089716

(57) **Abstract**

The position detection means detects a position of an endoscope based on an endoscopic image or the like. The speed estimation means estimates a withdrawal speed of the endoscope based on the acquired position of the endoscope. The display control means displays information indicating a region of interest, which is a region where the withdrawal speed was equal to or higher than a predetermined threshold value, on a display device based on the position of the endoscope and the withdrawal speed.

## Description

### TECHNICAL FIELD

The present disclosure relates to processing of images relating to an endoscopic examination.

### BACKGROUND ART

Conventionally, when carrying out an endoscopic examination, it is said to be preferable to remove the endoscope within the organ to be examined while taking a predetermined time. From this point of view, Patent Document 1 proposes a method that analyzes the variation in the movement speed of the endoscope in the endoscope system and, when it is presumed that the examiner is overlooking the lesion site, notifies the examiner of it.

### PRECEDING TECHNICAL REFERENCES

### PATENT DOCUMENT

Patent Document 1: International Publication WO2020/110214

### SUMMARY

### PROBLEM TO BE SOLVED

However, according to Patent Document 1, when the examiner checks the examination data after the endoscopic examination, he or she can not always refer to the information on the region where the withdrawal speed was fast in a suitable manner.

It is an object of the present disclosure to provide an information display device capable of referring to information relating to a region where the withdrawal speed was high, after the endoscopic examination.

### MEANS FOR SOLVING THE PROBLEM

According to an example aspect of the present disclosure, there is provided an information display device comprising:
a position detection means configured to detect a position of an endoscope;
a speed estimation means configured to estimate a withdrawal speed of the endoscope based on the position of the endoscope; and
a display control means configured to display information indicating a region of interest, which is a region where the withdrawal speed was equal to or higher than a predetermined threshold value, on a display device based on the position of the endoscope and the withdrawal speed.

According to another example aspect of the present disclosure, there is provided an information display method comprising:
detecting a position of an endoscope;
estimating a withdrawal speed of the endoscope based on the position of the endoscope; and
displaying information indicating a region of interest, which is a region where the withdrawal speed was equal to or higher than a predetermined threshold value, on a display device based on the position of the endoscope and the withdrawal speed.

According to still another example aspect of the present disclosure, there is provided a recording medium storing a program, the program causing a computer to execute processing of:
detecting a position of an endoscope;
estimating a withdrawal speed of the endoscope based on the position of the endoscope; and
displaying information indicating a region of interest, which is a region where the withdrawal speed was equal to or higher than a predetermined threshold value, on a display device based on the position of the endoscope and the withdrawal speed.

### EFFECT

According to the present disclosure, after the endoscopic examination, the examination result can be checked mainly in an area where the withdrawal speed was fast, and it is possible to efficiently determine whether or not to perform a re-examination.

### BRIEF DESCRIPTION OF THE DRAWINGS

[FIG. 1] FIG. 1 is a block diagram showing a schematic configuration of an endoscopic examination system.
[FIG. 2] FIG. 2 is a block diagram showing a hardware configuration of an image processing device.
[FIG. 3] FIG. 3 is a block diagram showing a functional configuration of the image processing device.
[FIG. 4] FIG. 4 schematically shows a structure of a large intestine.
[FIG. 5] FIG. 5 shows a display example of an under-examination screen mode.
[FIG. 6] FIG. 6 shows a display example of an examination result screen mode.
[FIG. 7] FIG. 7 shows another display example of the examination result screen mode.
[FIG. 8] FIG. 8 shows still another display example of the examination result screen mode.
[FIG. 9] FIG. 9 shows still another display example of the examination result screen mode.
[FIG. 10] FIG. 10 is a flowchart of display processing by an image processing device.
[FIG. 11] FIG. 11 is a block diagram showing a functional configuration of an information display device of a second example embodiment.
[FIG. 12] FIG. 12 is a flowchart of processing by the information display device of the second example embodiment.

### EXAMPLE EMBODIMENTS

Preferred example embodiments of the present disclosure will be described with reference to the accompanying drawings.

### <First example embodiment>

### [System Configuration]

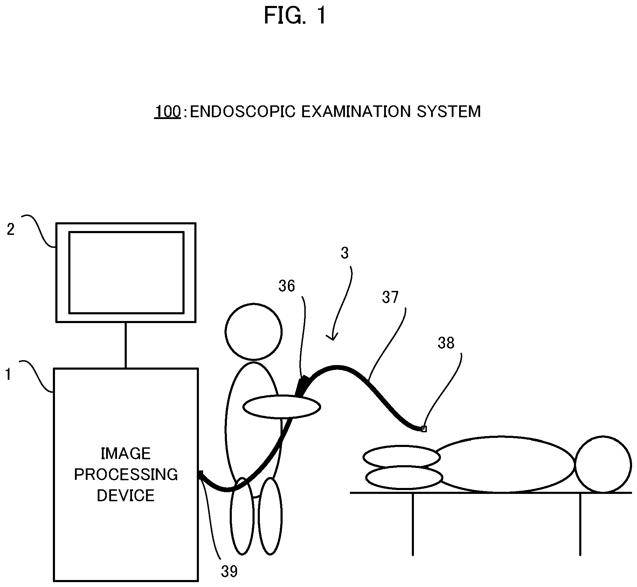

FIG. 1 shows a schematic configuration of an endoscopic examination system 100. The endoscopic examination system 100 detects the position of the endoscope and estimates the withdrawal speed of the endoscope based on the position at the time of examination (including treatment) using the endoscope. Then, the endoscopic examination system 100 determines whether or not the withdrawal speed is equal to or higher than a predetermined threshold value and displays the result. In addition, the endoscopic examination system 100 may detect lesions by AI image analysis at the time of examination (including treatment) using the endoscope and display the images together with the above-described result. This makes it possible to easily grasp the regions where the withdrawal speed was high or where lesions were detected, after the endoscopic examination.

As shown in FIG. 1, the endoscopic examination system 100 mainly includes an image processing device 1, a display device 2, and an endoscope 3 connected to the image processing device 1.

The image processing device 1 acquires a moving image (hereinafter also referred to as an "endoscopic image Ic") captured by the endoscope 3 during the endoscopic examination from the endoscope 3 and displays display data for the check by the examiner of the endoscopic examination on the display device 2. Specifically, the image processing device 1 acquires a moving image of organs captured by the endoscope 3 as an endoscopic image Ic during the endoscopic examination. In addition, when the examiner finds a lesion during the endoscopic examination, he or she operates the endoscope 3 to input a photographing instruction of the lesion position. Based on the photographing instruction by the examiner, the image processing device 1 generates a lesion image capturing the lesion position. Specifically, the image processing device 1 generates the lesion image which is a still image, from the endoscopic image Ic which is a moving image, on the basis of the photographing instruction of the examiner.

The display device 2 is a display or the like for displaying images on the basis of the display signal supplied from the image processing device 1.

The endoscope 3 mainly includes an operation unit 36 used by the examiner to input instructions such as air supply, water supply, angle adjustment, and the photographing instruction, a shaft 37 having flexibility and inserted into an organ of a subject to be examined, a tip portion 38 with a built-in image-taking unit such as an ultra-compact imaging element, and a connection unit 39 for connection with the image processing device 1.

While the following explanation is mainly given on the processing of endoscopic examination for a large intestine, the subjects of examination may be gastrointestinal (digestive organs) such as the stomach, esophagus, small intestine, and duodenum, as well as the large intestine.

### [Hardware configuration]

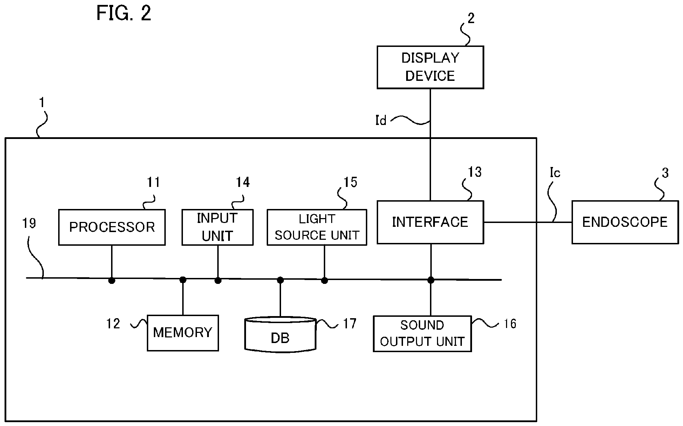

FIG. 2 shows a hardware configuration of the image processing device 1. The image processing device 1 mainly includes a processor 11, a memory 12, an interface 13, an input unit 14, a light source unit 15, a sound output unit 16, and a data base (hereinafter referred to as "DB") 17. These elements are connected with each other via a data bus 19.

The processor 11 executes a predetermined processing by executing a program stored in the memory 12. The processor 11 is a processor such as a CPU (Central Processing Unit), a GPU (Graphics Processing Unit), and a TPU (Tensor Processing Unit). The processor 11 may be configured by multiple processors. The processor 11 is an example of a computer.

The memory 12 is configured by various volatile memories used as a working memory and non-volatile memories for storing information needed for the processing of the image processing device 1, such as a RAM (Random Access Memory) and a ROM (Read Only Memory). Incidentally, the memory 12 may include an external storage device such as a hard disk connected to or incorporated in the image processing device 1, and may include a storage medium such as a removable flash memory or a disk medium. The memory 12 stores a program for the image processing device 1 to execute processing in the present example embodiment.

Also, the memory 12 temporarily stores a series of endoscopic videos Ic taken by the endoscope 3 in the endoscopic examination, based on the control of the processor 11. Further, the memory 12 temporarily stores the lesion images photographed in response to the photographing instructions by the examiner during the endoscopic examination. These images are stored in the memory 12 in association with, for example, subject identification information (e.g., the patient ID) and time stamp information, etc.

The interface 13 performs an interface operation between the image processing device 1 and the external devices. For example, the interface 13 supplies the display data Id generated by the processor 11 to the display device 2. Also, the interface 13 supplies the illumination light generated by the light source unit 15 to the endoscope 3. Also, the interface 13 supplies an electrical signal indicating the endoscopic image Ic supplied from the endoscope 3 to the processor 11. The interface 13 may be a communication interface such as a network adapter for wired or wireless communication with an external device, or may be a hardware interface compliant with a USB (Universal Serial Bus), SATA (Serial Advanced Technology Attachment), etc.

The input unit 14 generates an input signal based on the operation of the examiner. The input unit 14 is, for example, a button, a touch panel, a remote controller, a voice input device, or the like. The light source unit 15 generates the light to be delivered to the tip portion 38 of the endoscope 3. The light source unit 15 may also incorporate a pump or the like for delivering water or air to be supplied to the endoscope 3. The sound output unit 16 outputs the sound based on the control of the processor 11.

The DB 17 stores the endoscopic images acquired by past endoscopic examinations of the subject, and the examination result information. The DB 17 may include an external storage device, such as a hard disk connected to or incorporated in the image processing device 1, and may include a storage medium, such as a removable flash memory. Instead of providing the DB 17 in the endoscopic examination system 100, the DB 17 may be provided in an external server or the like to acquire associated information from the server through communication.

### [Functional configuration]

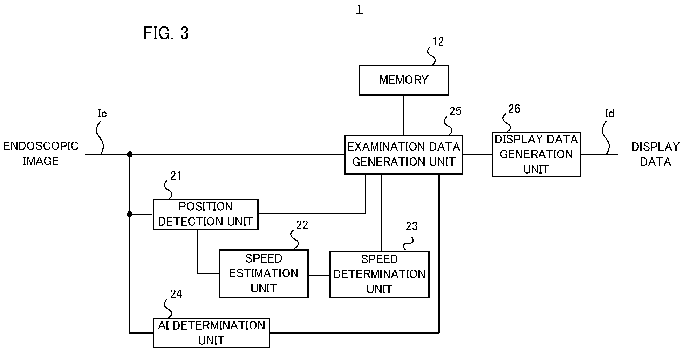

FIG. 3 is a block diagram showing a functional configuration of the image processing device 1. The image processing device 1 functionally includes a position detection unit 21, a speed estimation unit 22, a speed determination unit 23, an AI determination unit 24, an examination data generation unit 25, and a display data generation unit 26.

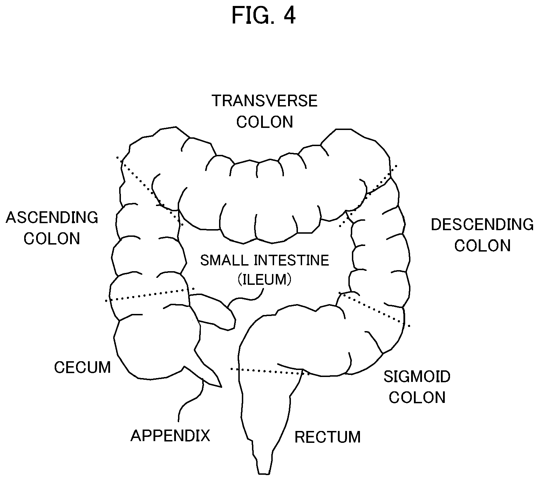

The image processing device 1 receives the endoscopic image Ic from the endoscope 3. The endoscopic image Ic is inputted into the position detection unit 21, the AI determination unit 24, and the examination data generation unit 25. The position detection unit 21 detects the position of the endoscope 3, i.e., the imaging position of the endoscopic image, based on the endoscopic image Ic. Specifically, the position detection unit 21 detects the imaging position by image analysis of the inputted endoscopic image Ic. Here, the imaging position may be three-dimensional coordinates in the organ of the examination target, but may be at least an information indicating one of a plurality of regions in the organ of the examination target. For example, as shown in FIG. 4, the large intestine includes multiple regions (sites) such as the cecum, the ascending colon, the transverse colon, the descending colon, the sigmoid colon, and the rectum. Therefore, when the examination target is the large intestine, the position detection unit 21 may detect whether at least the imaging position belongs to any of the above-described regions.

Specifically, the position detection unit 21 can estimate which region of the large intestine the imaging position at that time belongs to, based on the pattern of the mucous membrane, the presence or absence of the folds, the shape of the folds in the endoscopic image, the number of the folds passed by the movement of the endoscope 3, and the like. In addition to the image analysis of the endoscopic image, the position detection unit 21 may detect the imaging position using the insertion length of the endoscope 3 inserted into the organ. In the present example embodiment, the detection method of the imaging position in the organ of the examination target is not limited to a specific method. The position detection unit 21 outputs the detected imaging position to the speed estimation unit 22 and the examination data generation unit 25.

The speed estimation unit 22 estimates the withdrawal speed of the endoscope based on the imaging positions detected by the position detection unit 21. When the position detection unit 21 cannot detect the three-dimensional coordinates of the imaging position, the speed estimation unit 22 may estimate the withdrawal speed of the endoscope by the image analysis of the taken images, and may estimate the withdrawal speed of the endoscope based on the change amount of the insertion length of the endoscope 3. The speed estimation unit 22 may also estimate the withdrawal speed of the endoscope by combining these methods. The speed estimation unit 22 outputs the estimated withdrawal speed to the speed determination unit 23.

The speed determination unit 23 determines whether or not the withdrawal speed estimated by the speed estimation unit 22 is equal to or higher than a predetermined threshold value. The predetermined threshold value is a value determined in advance based on the examination time recommended in the guideline of the endoscopic examination. The speed determination unit 23 outputs the determination result to the examination data generation unit 25.

The AI determination unit 24 performs image analysis on the endoscopic image Ic to determine whether or not lesions exist. The AI determination unit 24 detects lesion-like parts included in the endoscopic image using an image recognition model prepared in advance. When detecting the lesion-like part, the AI determination unit 24 outputs the position (lesion position) and a score indicating probability of lesion as a determination result. On the other hand, when the AI determination unit does not detect any lesion-like part, it outputs the determination result indicating that there is no lesion. The AI determination unit 24 outputs the determination result to the examination data generation unit 25.

The examination data generation unit 25 generates the examination data on the basis of the endoscopic image Ic, the imaging position detected by the position detection unit 21, the determination result of the withdrawal speed determined by the speed determination unit 23, and the determination result of the presence or absence of the lesion determined by the AI determination unit 24, and temporarily stores the generated examination data in the memory 12. Specifically, when the examiner operates the endoscope 3, the examination data generation unit 25 generates the examination data including the endoscopic image Ic taken by the endoscope 3, the basic information such as the patient name, the patient ID, and the examination date and time, the determination result of the withdrawal speed at the imaging position, and the determination result of the presence or absence of the lesion at the imaging position, and stores the examination data in the memory 12. The patient ID is information that uniquely identifies the patient, and may be an ID uniquely assigned to each patient or a personal identification number such as a My Number. The examination data generation unit 25 outputs the generated examination data to the display data generation unit 26.

The display data generation unit 26 generates the display data Id using the examination data inputted from the examination data generation unit 25, and outputs the display data to the display device 2. The display data generation unit 26 includes, as main screen display modes, an under-examination screen mode and an examination result screen mode. Here, the "under-examination screen mode" is a display mode for the examiner to check, in real time, the endoscopic image Ic taken by the endoscope 3 during the endoscopic examination. In addition, the "examination result screen mode" is a mode for displaying the result of the endoscopic examination. The examination result screen mode is used, for example, to check for missing regions in the endoscopic examination of the patient.

### [Display example]

Next, a display example by the display device 2 will be described.

### (Under-examination screen mode)

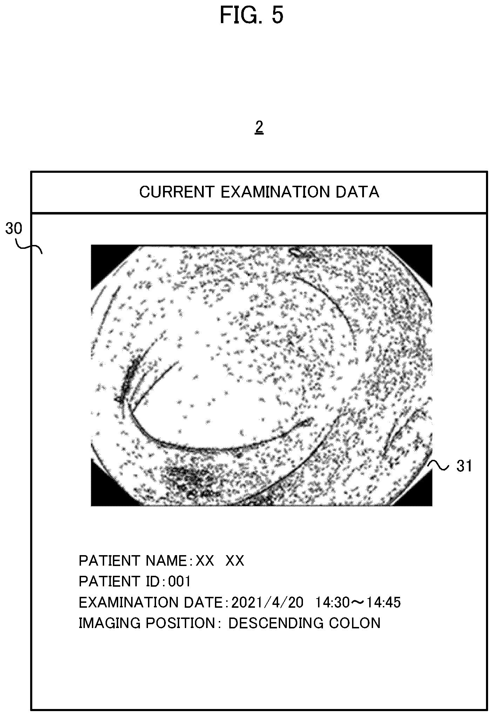

FIG. 5 shows a display example of the under-examination screen mode. This example is a display example of the display device 2 during the endoscopic examination of a certain patient. Specifically, in the display example of FIG. 5, the image 31 of the current imaging position is displayed in the display area 30 in addition to the information such as the patient name, the patient ID, the examination date and time, and the current imaging position.

### (Examination result screen mode)

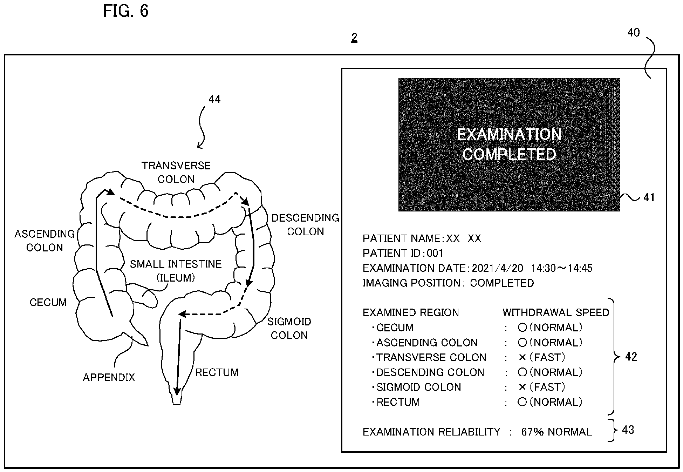

FIG. 6 shows a display example of the examination result screen mode. This example is a display example of the display device 2 after the endoscopic examination of a certain patient is completed.

Specifically, in the display example of FIG. 6, in the display area 40, the patient name, the endoscopic image 41, the withdrawal speed determination result 42, and the examination reliability 43 are displayed in addition to the patient ID, the examination date and time, and the imaging position. Further, the model image 44 is displayed outside the display area 40. Since this example is a display example after the examination, the "EXAMINATION COMPLETED" image representing that the examination is completed is displayed as the endoscopic image 41. Instead of the above "EXAMINATION COMPLETED" image, the image of the lesion detected during the examination may be displayed.

The withdrawal speed determination result 42 shows the determination result of whether or not the withdrawal speed in each region of the organ was equal to or higher than the predetermined threshold value. Here, as an example, the withdrawal speed is displayed as " O (NORMAL)" when it is lower than the predetermined threshold value, and " × (FAST)" when it is equal to or higher than the predetermined threshold value. The region in which the withdrawal speed was equal to or higher than the predetermined threshold value is also referred to as a "region of interest".

The examination reliability 43 shows the examination reliability based on the withdrawal speed. Here, as the examination reliability, the ratio of the regions (the cecum, the ascending colon, the descending colon and the rectum in this example) where the withdrawal speed was lower than the predetermined threshold value to the entire organ (the cecum, the ascending colon, the transverse colon, the descending colon, the sigmoid colon and the rectum in this example) is displayed. Instead of displaying the above-described ratio itself, the reliability level of five stages (reliability 1 to 5, reliability 5 is the most reliable) may be determined and displayed based on the above-described ratio.

The model image 44 is a model image of the organ of the examination target (the large intestine in the present example embodiment), on which the determination result of the withdrawal speed is displayed in an overlapping manner. Here, for convenience of illustration, the regions where the withdrawal speed was normal (the cecum, the ascending colon, the descending colon, and the rectum in this example) are represented by the solid line, and the regions where the withdrawal speed was fast (the transverse colon and the sigmoid colon in this example) are represented by the broken line. Instead of the solid line and the broken line, they may be distinguished by the thickness of the line, the color of the line, or else.

By displaying the examination result screen mode as described above, the examiner or the like can easily grasp the accuracy of the examination and the regions where the withdrawal speed was fast. Therefore, it becomes possible to appropriately determine the treatment to be performed in the future.

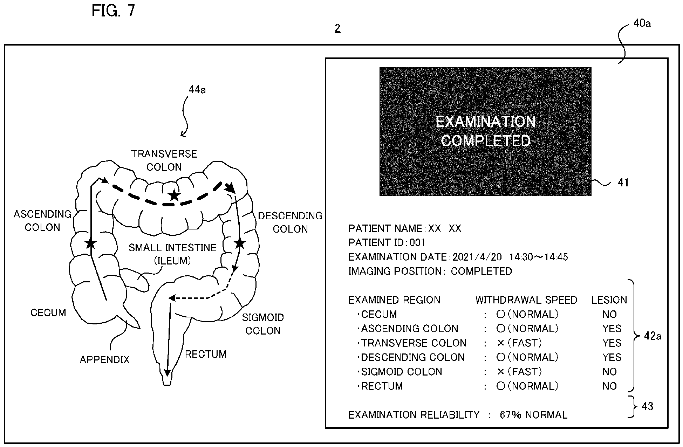

FIG. 7 shows another display example of the examination result screen mode. This example is an example in the case of adding the determination result of the presence or absence of the lesion to the display example of FIG. 6.

Specifically, in the display example of FIG. 7, the determination result of the presence or absence of the lesion is displayed in the withdrawal speed determination result 42a in the display area 40a and the model image 44a. In the withdrawal speed determination result 42a, the determination result of the presence or absence of the lesion in each region of the organ is displayed in addition to the determination result of whether or not the withdrawal speed in each region of the organ was equal to or higher than the predetermined threshold. Here, for example, for the region in which the AI determination unit determines that there is a lesion, "YES" is displayed in the item of "LESION", and for the region in which the AI determination unit determines that there is no lesion, "NO" is displayed in the item of "LESION".

In the model image 44a, in addition to the display of the determination results of the withdrawal speed (i.e., the solid line or the broken line) , a star mark is displayed in the region where the AI determination unit has determined that there is a lesion. Specifically, the regions where the withdrawal speed was normal (the cecum, the ascending colon, the descending colon, and the rectum in this example) are represented by the solid lines. In addition, the region where there is a lesion (star mark) and the withdrawal speed was fast (the transverse colon in this example) is represented by the thick dashed line. In addition, the region where there is no lesion (star mark) and the withdrawal speed was fast (the sigmoid colon in this example) is represented by a thin broken line. Thus, in the display example of FIG. 7, the region where simply the withdrawal speed was fast is distinguished from the region where the withdrawal speed was fast and the lesion is found. This makes it possible for the examiner or the like to easily grasp the region where there is a lesion and the withdrawal speed was fast. Thus, it is possible to preferentially check the region where there is a high possibility of overlooking the lesion.

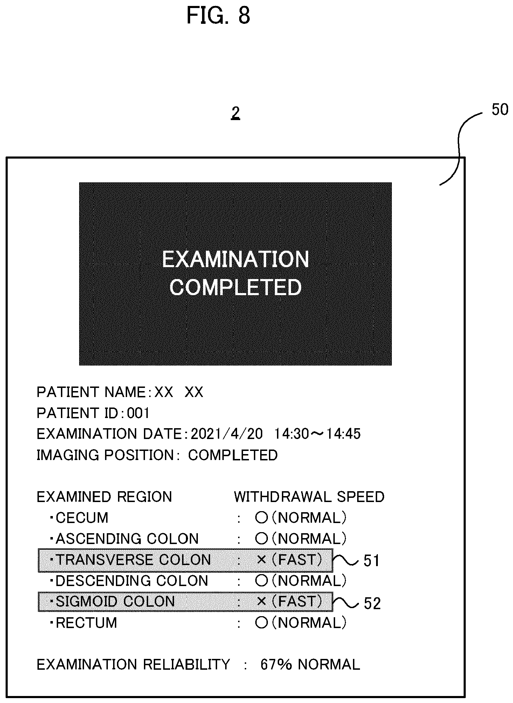

FIG. 8 shows still another display example of the examination result screen mode. In this example, the model image of the organ is omitted from the example of FIG. 6. Specifically, the information displayed on the display area 50 of FIG. 8 is similar to the information displayed on the display area 40 of FIG. 6. In addition to this, in FIG. 8, the rows of the regions in which the determination result of the withdrawal speed is displayed as "× (FAST)" is surrounded by the rectangles 51 and 52. Thus, even without the model image, it is possible to easily grasp the regions where the withdrawal speed was fast.

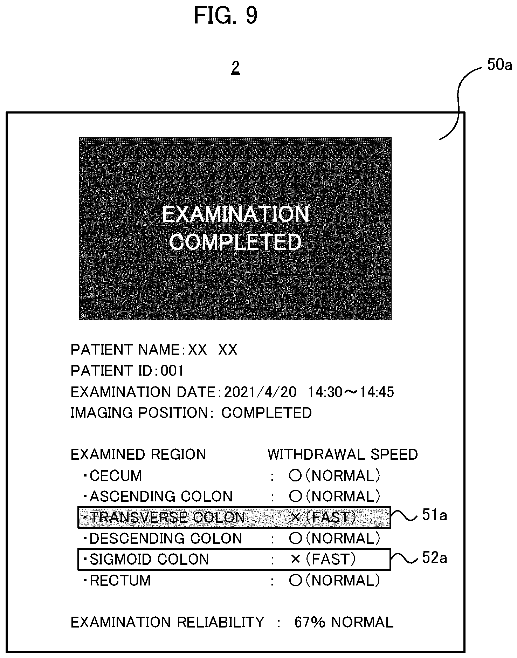

FIG. 9 shows still another display example of the examination result screen mode. In this example, the model image of the organ is omitted from the example of FIG. 7. Specifically, the information displayed on the display area 50a of FIG. 9 is similar to the information displayed on the display area 40a of FIG. 7. In addition to this, in FIG. 9, the row of the region in which the lesion was found and the determination result of the withdrawal speed is displayed as "× (FAST)" are surrounded by a gray rectangle 51a. In addition, the row of the region in which no lesion was found and the determination result of the withdrawal speed displayed as " × (FAST)" is surrounded by a white rectangle 52a. Thus, even without the model image, it is possible to easily grasp the region where the lesion was found and the withdrawal speed was fast.

### [Display processing]

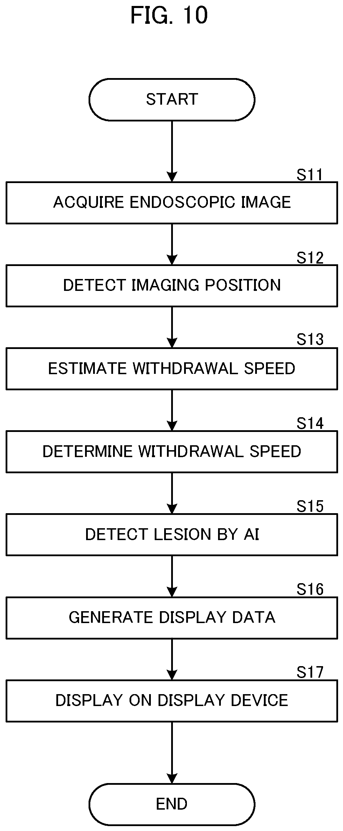

Next, display processing for performing the above-mentioned display will be described. FIG. 10 is a flowchart of display processing by the image processing device 1. This processing is realized by the processor 11 shown in FIG. 2, which executes a pre-prepared program and operates as each element shown in FIG. 3. Incidentally, FIG. 10 is an example in the case of displaying the display example of FIG. 7.

First, the position detection unit 21, the AI determination unit 24 and the examination data generation unit 25 acquire the endoscopic image Ic from the endoscope 3 (step S11). Next, the position detection unit 21 detects the imaging position based on the endoscopic image Ic (step S12). Next, the speed estimation unit 22 estimates the withdrawal speed of the endoscope using the detected imaging position or the like (step S13). Next, the speed determination unit 23 determines whether or not the withdrawal speed acquired from the speed estimation unit 22 is equal to or higher than a predetermined threshold value (step S14). Also, the AI determination unit 24 detects the presence or absence of a lesion from the endoscopic image Ic (step S15). Incidentally, step S15 may be performed prior to step S12, or may be performed simultaneously with steps S12 to S14.

Next, the display data generation unit 26 acquires the examination data including the endoscopic image 41, the basic information such as the patient name, the patient ID, and the examination date and time, the determination result of the withdrawal speed at the imaging position, the determination result of the presence or absence of a lesion at the imaging position, and the examination reliability, as shown in FIG. 7, from the examination data generation unit 25, and generates and outputs the display data as illustrated in FIG. 7 to the display device 2 (step S16). The display device 2 displays the received display data (step S17). Thus, the display like the display example of FIG. 7 is performed.

### <Second example embodiment>

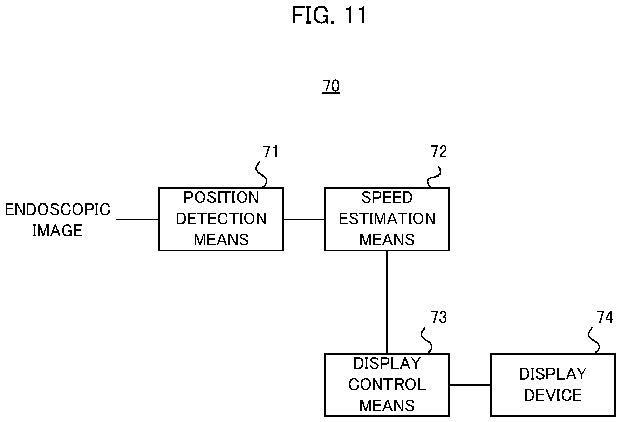

FIG. 11 is a block diagram showing a functional configuration of an information display device of a second example embodiment. The information display device 70 includes a position detection means 71, a speed estimation means 72, a display control means 73, and a display device 74.

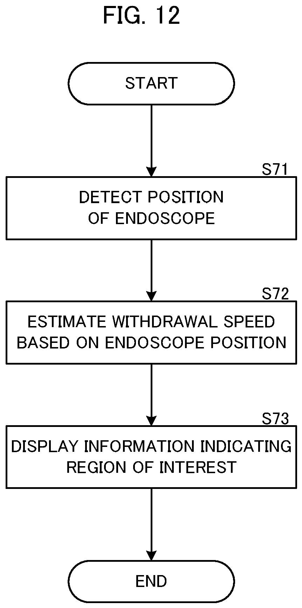

FIG. 12 is a flowchart of processing by the information display device of the second example embodiment. The position detection means 71 detects a position of an endoscope based on an endoscopic image or the like (step S71). The speed estimation means 72 estimates a withdrawal speed of the endoscope based on the acquired position of the endoscope (step S72). The display control means 73 displays information indicating a region of interest, which is a region where the withdrawal speed was equal to or higher than a predetermined threshold value, on a display device 74 based on the position of the endoscope and the withdrawal speed (step S73).

According to the information display device 70 of the second example embodiment, it is possible to check the examination result centering on the region where the withdrawal speed was fast, after the endoscopic examination.

A part or all of the above example embodiments may also be described as in the following supplementary notes, but are not limited to:

### (Supplementary note 1)

An information display device comprising:
a position detection means configured to detect a position of an endoscope;
a speed estimation means configured to estimate a withdrawal speed of the endoscope based on the position of the endoscope; and
a display control means configured to display information indicating a region of interest, which is a region where the withdrawal speed was equal to or higher than a predetermined threshold value, on a display device based on the position of the endoscope and the withdrawal speed.

### (Supplementary note 2)

The information display device according to Supplementary note 1, further comprising a lesion detection means configured to perform an image analysis on a captured image taken by the endoscope to determine presence or absence of the lesion,
wherein the display control means displays a region where the withdrawal speed was equal to or higher than the predetermined threshold value and the lesion is detected by the image analysis, as the region of interest.

### (Supplementary note 3)

The information display device according to Supplementary note 2, wherein the display control means displays a region where the withdrawal speed was equal to or higher than the predetermined threshold value and the lesion is detected by the image analysis, in a manner distinguishable from a region where the withdrawal speed was lower than the predetermined threshold value.

### (Supplementary note 4)

The information display device according to any one of Supplementary notes 1 to 3, wherein the display control means superimposes and displays the region of interest on a model image indicating an entire organ of an examination target.

### (Supplementary note 5)

The information display device according to any one of Supplementary notes 1 to 3, wherein the display control means displays the region of interest by characters in units of regions forming the organ of the examination target.

### (Supplementary note 6)

The information display device according to any one of Supplementary notes 1 to 5, wherein the display control means displays information indicating a ratio of the regions where the withdrawal speed was equal to or higher than the predetermined threshold value to the entire organ of the examination target.

### (Supplementary note 7)

The information display device according to any one of Supplementary notes 1 to 6, wherein the display control means displays the region of interest after an examination using the endoscope is completed.

### (Supplementary note 8)

An information display method comprising:
detecting a position of an endoscope;
estimating a withdrawal speed of the endoscope based on the position of the endoscope; and
displaying information indicating a region of interest, which is a region where the withdrawal speed was equal to or higher than a predetermined threshold value, on a display device based on the position of the endoscope and the withdrawal speed.

### (Supplementary note 9)

A recording medium storing a program, the program causing a computer to execute processing of:
detecting a position of an endoscope;
estimating a withdrawal speed of the endoscope based on the position of the endoscope; and
displaying information indicating a region of interest, which is a region where the withdrawal speed was equal to or higher than a predetermined threshold value, on a display device based on the position of the endoscope and the withdrawal speed.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

### DESCRIPTION OF SYMBOLS

1 Image processing device
2 Display device
3 Endoscope
11 Processor
12 Memory
17 Database (DB)
21 Position detection unit
22 Speed estimation unit
23 Speed determination unit
24 AI determination unit
25 Examination data generation unit
26 Display data generation unit
100 Endoscopic examination system

## Claims

1. An information display device comprising:
a position detection means configured to detect a position of an endoscope;
a speed estimation means configured to estimate a withdrawal speed of the endoscope based on the position of the endoscope; and
a display control means configured to display information indicating a region of interest, which is a region where the withdrawal speed was equal to or higher than a predetermined threshold value, on a display device based on the position of the endoscope and the withdrawal speed.

2. The information display device according to claim 1, further comprising a lesion detection means configured to perform an image analysis on a captured image taken by the endoscope to determine presence or absence of the lesion,
wherein the display control means displays a region where the withdrawal speed was equal to or higher than the predetermined threshold value and the lesion is detected by the image analysis, as the region of interest.

3. The information display device according to claim 2, wherein the display control means displays a region where the withdrawal speed was equal to or higher than the predetermined threshold value and the lesion is detected by the image analysis, in a manner distinguishable from a region where the withdrawal speed was lower than the predetermined threshold value.

4. The information display device according to any one of claims 1 to 3, wherein the display control means superimposes and displays the region of interest on a model image indicating an entire organ of an examination target.

5. The information display device according to any one of claims 1 to 3, wherein the display control means displays the region of interest by characters in units of regions forming the organ of the examination target.

6. The information display device according to any one of claims 1 to 5, wherein the display control means displays information indicating a ratio of the regions where the withdrawal speed was equal to or higher than the predetermined threshold value to the entire organ of the examination target.

7. The information display device according to any one of claims 1 to 6, wherein the display control means displays the region of interest after an examination using the endoscope is completed.

8. An information display method comprising:
detecting a position of an endoscope;
estimating a withdrawal speed of the endoscope based on the position of the endoscope; and
displaying information indicating a region of interest, which is a region where the withdrawal speed was equal to or higher than a predetermined threshold value, on a display device based on the position of the endoscope and the withdrawal speed.

9. A recording medium storing a program, the program causing a computer to execute processing of:
detecting a position of an endoscope;
estimating a withdrawal speed of the endoscope based on the position of the endoscope; and
displaying information indicating a region of interest, which is a region where the withdrawal speed was equal to or higher than a predetermined threshold value, on a display device based on the position of the endoscope and the withdrawal speed.
